# EUROPEAN PATENT SPECIFICATION

(11) **EP 3 971 574 B1**
(45) Date of publication and mention of the grant of the patent: **07.05.2025**
(21) Application number: 19937668.2
(22) Date of filing: 15.07.2019
(51) Int. Cl.: G01N 33/80, G01N 33/543

(54) **FUNCTIONALIZED BIOLOGICAL MULTILAYER POROUS MEMBRANE IMMUNE CARRIER, PREPARATION METHOD, AND APPLICATION**
FUNKTIONALISIERTER BIOLOGISCHER MEHRSCHICHTMEMBRAN-IMMUNTRÄGER, HERSTELLUNGSVERFAHREN UND ANWENDUNG
ÉLÉMENT PORTEUR IMMUNITAIRE À MEMBRANE POREUSE MULTICOUCHE BIOLOGIQUE FONCTIONNALISÉ, PROCÉDÉ DE PRÉPARATION, ET APPLICATION

(43) Date of publication of application: 23.03.2022
(73) Proprietor: Suzhou Institute of Biomedical Engineering and Technology Chinese Academy of Sciences, Suzhou, Jiangsu 215163 (CN)
(72) Inventor: LI, Yong, Changchun, Jilin 130000 (CN); TANG, Yuguo, Changchun, Jilin 130000 (CN); WANG, Hongmei, Suzhou, Jiangsu 215163 (CN); DUAN, Shengbao, Suzhou, Jiangsu 215163 (CN); DING, Shaohua, Suzhou, Jiangsu 215000 (CN); CHEN, Yezhou, Suzhou, Jiangsu 215000 (CN); WEI, Shuangshi, Suzhou, Jiangsu 215000 (CN); LI, Jianping, Shenyang, Liaoning 110000 (CN)
(74) Representative: Puchberger & Partner Patentanwälte
(86) International application number: PCT/CN2019/095958
(87) International publication number: WO 2021/007738

(56) References cited:
- WO-A1-2006/004332
- AU-B2- 610 818
- CN-A- 102 901 828
- CN-A- 112 684 171
- US-A- 4 275 053
- US-A- 4 275 053
- US-A- 4 661 445
- QIANG LU ET AL: "Surface immobilization of antibody on silk fibroin through conformational transition", ACTA BIOMATERIALIA, ELSEVIER, AMSTERDAM, NL, vol. 7, no. 7, 2 March 2011 (2011-03-02), pages 2782 - 2786, XP028222108, ISSN: 1742-7061, [retrieved on 20110305], DOI: 10.1016/J.ACTBIO.2011.03.001
- THEN W. L. ET AL: "The detection of blood group phenotypes using paper diagnostics", VOX SANGUINIS, vol. 108, no. 2, 9 September 2014 (2014-09-09), CH, pages 186 - 196, XP055915952, ISSN: 0042-9007, DOI: 10.1111/vox.12195
- WANG HONGMEI ET AL: "Silk cocoon membrane-based immunosensing assay for red blood cell antigen typing", SENSORS AND ACTUATORS B: CHEMICAL, ELSEVIER BV, NL, vol. 320, 30 May 2020 (2020-05-30), XP086242378, ISSN: 0925-4005, [retrieved on 20200530], DOI: 10.1016/J.SNB.2020.128376

## Description

### TECHNICAL FIELD

The invention relates to the field of immunodetection, and particularly relates to a functionalized biological multilayer porous membrane immune carrier, a preparation method and use.

### BACKGROUD

Incompatible blood groups between a blood donor and a blood recipient can cause a severe hemolytic transfusion reaction, one of the most important approaches for ensuring safe blood transfusion is blood group compatibility detection, compatibility, i.e., harmonious coexistence, refers to inexistence of a specific antibody corresponding to an input red blood cell (RBC) antigen in a serum of a patient, and nonoccurrence of an immune reaction, which is called as compatible blood transfusion. On the contrary, it is called as incompatible blood transfusion, i.e., there is the specific antibody corresponding to the input RBC antigen in the serum of the patient, the immune reaction is performed, and RBCs input into the body of the patient are damaged, resulting in hemolysis. A specific antigen on the surfaces of the RBCs and an antibody in the serum determine a blood group, and blood group compatibility detection before blood transfusion includes ABO forward and reverse typing, RhD blood group typing, detection on an irregular antibody in the serum of the blood recipient, detection and identification of a clinically significant antibody, a crossed matching test and the like.

The most conventional gold standard detection method for blood group detection is a test tube method, and the test tube method needs the cumbersome centrifuging and washing steps and preparation of a cell suspension. Other conventional blood group detection methods include a slide method, micrcolumn gel immunoassay (MGIA), a micropore plate solid-phase detection method and the like. The slide detection technology is a cheap and rapid blood group method. However, this method is not sensitive enough to detect a weak antigen subgroup, and in this method, only forward typing detection can be carried out. In the MGIA, forward typing and reverse typing detection can be carried out, and the washing step can be avoided, but fibrinogens or obvious white blood cells are increased in a to-be-detected sample, resulting in a false positive result, and meanwhile, the incubating and centrifuging steps are required, and thus, MGIA is not applicable to undeveloped areas and particularly most of developing countries in which it is inconvenient for the MGIA to carry out bedside blood group detection. In addition, the high price monopoly and the limited detection flux of a raw material, i.e., gel particles, are also disadvantage factors of limiting further wide application of MGIA to clinic and a blood station. The micropore plate solid-phase detection method improves detection sensitivity, but also needs the complex incubating and washing steps. In addition to the above methods, fiber filter paper has the fiber characteristics of filtering out the RBCs and reserving and agglutinating the RBCs, and thus, a method for detecting the blood group by the filter paper is widely reported. The filter paper detection technology mainly adopts physical adsorption to fix the antibody onto the filter paper and is limited in adsorption capacity, and thus is relatively low in detection sensitivity and is not widely applied clinically. In addition, an IgG type monoclonal antibody cannot be used on such fiber paper as a fixed antibody, because even though there is a positive reaction, but agglutination is not carried out, the RBCs to which the positive reaction occurs will still be washed away, resulting in a false positive reaction, and meanwhile, due to limitation by the preservation condition of the antibody, the IgG type monoclonal antibody is not applicable to field emergency conditions.

QIANG LU ET AL: "Surface immobilization of antibody on silk fibroin through conformational transition", ACTA BIOMATERIALIA, ELSEVIER, AMSTERDAM, NL, vol. 7, no. 7, 2 March 2011 (2011-03-02), pages 2782-2786, discloses a procedure for the immobilization of antibodies on silk fibroin substrates. By controlling the conformational transition of the silk fibroin, a primary antibody was immobilized and enriched at the surface of silk fibroin substrates under mild reaction conditions to maintain antibody function. Compared to chemical crosslinking, the immobilization efficiency in the present approach was increased significantly.

### SUMMARY

In order to overcome the defects in the prior art, one objective of the present invention is to provide a functionalized biological multilayer porous membrane immune carrier, including a biological multilayer porous membrane, a specific anti-biological multilayer porous membrane antibody attached to the surface of the biological multilayer porous membrane by antigen-antibody specific binding, and a cross-linked antibody coupled and bound with the specific anti-biological multilayer porous membrane antibody. The functionalized biological multilayer porous membrane immune carrier provided by the present invention can be bound to a RBC blood group antibody or a sensitized RBC blood group antigen by coupling or antibody-antigen specific binding so as to be further used for blood group detection; and the functionalized biological multilayer porous membrane immune carrier provided by the present invention can also select a corresponding specific capture antibody as required to perform immunoreactions or continue to carry out immunoselection and binding so as to be used for detecting and analyzing a pathogenic microbial cell antigen antibody, a bacterial cell antigen antibody, a tumor cell antigen antibody and various cell antigen antibodies.

The present invention provides a functionalized biological multilayer porous membrane immune carrier, including:
a biological multilayer porous membrane;
a specific anti-biological multilayer porous membrane antibody, the specific anti-biological multilayer porous membrane antibody being attached to the surface of the biological multilayer porous membrane through antigen-antibody specific binding with the biological multilayer porous membrane; and
a cross-linked antibody, the cross-linked antibody being coupled and bound with the specific anti-biological multilayer porous membrane antibody,
wherein the specific anti-biological multilayer porous membrane antibody is an antibody capable of undergoing an antigen-antibody specific reaction with the biological multilayer porous membrane; and
one Fab terminal of the cross-linked antibody is capable of being coupled with the specific anti-biological multilayer porous membrane antibody, and the other Fab terminal is capable of being bound to a specific antibody.

Preferably, the specific anti-biological multilayer porous membrane antibody includes a murine monoclonal antibody, a goat derived polyclonal antibody, a rabbit derived monoclonal antibody/polyclonal antibody or a chicken derived polyclonal antibody; the cross-linked antibody includes an anti-mouse polyclonal antibody, an anti-goat polyclonal antibody, an anti-rabbit polyclonal antibody or an anti-chicken polyclonal antibody; and the specific antibody which is capable of being bound to the other Fab terminal of the cross-linked antibody includes a murine monoclonal antibody, a goat derived polyclonal antibody, a rabbit derived monoclonal/polyclonal antibody or a chicken derived polyclonal antibody.

A second objective of the present invention is to provide a method for preparing the functionalized biological multilayer porous membrane immune carrier, including the following steps of:
S1: shearing cleaned thick cotton cloth or wool fabric or natural silk fabric or plant fiber fabric into a round or square membrane, then immersing the membrane into a treatment solution, and soaking for 2 to 4 hours in a water bath with a temperature of 40°C to 70°C to prepare a biological multilayer porous membrane; and
S2: carrying out antigen-antibody specific binding of the biological multilayer porous membrane with a specific anti-biological multilayer porous membrane antibody, washing with washing liquid, then coupling to a cross-linked antibody, washing with washing liquid, blocking with a blocking solution, and washing with washing liquid to prepare the functionalized biological multilayer porous membrane immune carrier as mentioned above,
wherein the specific anti-biological multilayer porous membrane antibody is an antibody capable of undergoing an antigen-antibody specific reaction with the biological multilayer porous membrane; and
one Fab terminal of the cross-linked antibody is capable of being coupled with the specific anti-biological multilayer porous membrane antibody, and the other Fab terminal is capable of being bound to a specific antibody.

Preferably, the specific anti-biological multilayer porous membrane antibody is diluted by a diluent to have a final concentration of 1 to 100µg/ml; and the cross-linked antibody is diluted by the diluent to have a final concentration of 1 to 100µg/ml, wherein the diluent is a phosphate buffer solution with a concentration of 0.01mol/L and a pH of 7.0 to 7.2.

Preferably, the treatment solution is a phosphate buffer solution with a concentration of 0.01mol/L and a pH of 7.0 to 7.2, which contains 1% bovine serum albumin.

Preferably, the washing liquid is a phosphate buffer solution with a concentration of 0.01mol/L and a pH of 7.0 to 7.2.

Preferably, the blocking solution is 5% skim milk powder prepared by a phosphate buffer solution with a concentration of 0.01mol/L and a pH of 7.0 to 7.2, wherein the unit % represents a mass of a solute in 100ml of solution.

A third objective of the present invention is to provide a biological multilayer porous membrane immune carrier for blood group compatibility detection, including:
the functionalized biological multilayer porous membrane immune carrier as mentioned above according to the present invention; and
a RBC blood group antibody or a sensitized RBC blood group antigen, attached to the functionalized biological multilayer porous membrane immune carrier through specific binding with the cross-linked antibody on the functionalized biological multilayer porous membrane immune carrier,
wherein the sensitized RBC blood group antigen is a RBC blood group antigen specifically bound to a RBC membrane antibody of a non-blood group antibody.

Preferably, the RBC blood group antibody includes antibodies of IgM type, IgG type or IgM-IgG mixed type for A, B, Rh, MNS, Kell and Kidd blood groups, and the red blood cell blood group antibody has a titer of greater than 1:32.

Preferably, the sensitized RBC blood group antigen includes a sensitized A-type RBC antigen, a sensitized B-type RBC antigen, a sensitized irregular antibody screening RBC antigen or a sensitized RBC panel cell antigen.

A fourth objective of the present invention is to provide a method for preparing a biological multilayer porous membrane immune carrier for blood group compatibility detection, including the following steps of:
S1: shearing cleaned thick cotton cloth or wool fabric or natural silk fabric or plant fiber fabric into a round or square membrane, then immersing the membrane into a treatment solution, and soaking for 2 to 4 hours in a water bath with a temperature of 40°C to 70°C to prepare a biological multilayer porous membrane;
S2: carrying out antigen-antibody specific binding to the biological multilayer porous membrane with a specific anti-biological multilayer porous membrane antibody, washing with washing liquid, then coupling to a cross-linked antibody, washing with washing liquid, blocking with a blocking solution, and washing with washing liquid to prepare the functionalized biological multilayer porous membrane immune carrier as mentioned above; and
S3: carrying out specific binding of a RBC blood group antibody or a sensitized RBC blood group antigen to the cross-linked antibody on the functionalized biological multilayer porous membrane immune carrier to prepare the biological multilayer porous membrane immune carrier for blood group compatibility detection as mentioned above,
wherein the specific anti-biological multilayer porous membrane antibody is an antibody capable of undergoing an antigen-antibody specific reaction with the biological multilayer porous membrane;
one Fab terminal of the cross-linked antibody is capable of being coupled with the specific anti-biological multilayer porous membrane antibody, and the other Fab terminal is capable of being bound to a specific antibody, and the specific antibody is a RBC blood group antibody or a sensitized RBC blood group antigen capable of being bound to the Fab terminal of the cross-linked antibody; and
the sensitized RBC blood group antigen is a RBC blood group antigen specifically bound to a RBC membrane antibody of a non-blood group antibody.

Preferably, the method for preparing the biological multilayer porous membrane immune carrier for blood group compatibility detection further includes a step of preparing the sensitized RBC blood group antigen, and the specific steps of preparing the sensitized RBC blood group antigen are as follows: reacting the RBC membrane antibody of the non-blood group antibody with fresh RBCs, lysing the RBCs by a hypotonic solution when the intensity of agglutination reaches 1+, centrifuging and collecting to obtain the sensitized RBC antigen.

Preferably, the hypotonic solution is a 0.1-0.7% NaCl solution.

Preferably, the centrifuging is carried out at 12,000 rpm for 5min.

A fifth objective of the present invention is to provide a blood group detection card, including:
a solid-phase plastic carrier, the solid-phase plastic carrier including a clamping groove outer shell and a clamping groove inner shell, the clamping groove outer shell and the clamping groove inner shell being in detachable connection;
the biological multilayer porous membrane immune carrier for blood group compatibility detection as mentioned above, positioned at the bottom of the clamping groove outer shell of the solid-phase plastic carrier;
an absorbent cotton, positioned at the bottom of the clamping groove inner shell of the solid-phase plastic carrier; and
an isolating membrane, arranged above the absorbent cotton.

Preferably, the solid-phase plastic carrier is made of a material selected from polyethylene, polystyrene and styrene-butadiene-acrylonitrile; the solid-phase plastic carrier further includes a detection hole, including two holes, three holes or multiple holes; the isolating membrane includes a filter paper, cellulose fibers, a glass fiber mat, a fiber mat made of a porous high polymer material or a test paper; and the absorbent cotton includes a cotton pulp mat and a filter paper.

A sixth objective of the present invention is to provide a method for detecting a blood group, including the following steps of:
adding a to-be-detected blood group sample into the detection hole of the blood group detection card as mentioned above, and allowing the to-be-detected blood group sample to stand for at least 1 minute; and rinsing with washing liquid, observing the color development of the detection hole, wherein red color development indicates a positive result, and a natural color development indicates a negative result,
wherein a positive result is observed if the to-be-detected blood group sample undergoes an antigen-antibody specific reaction with the biological multilayer porous membrane immune carrier for blood group compatibility detection in the blood group detection card, and otherwise, the result is negative.

Preferably, the washing liquid is a buffer solution containing 0.05% to 0.5% of Tween-20, the pH value of the washing liquid is 7.0 to 7.2, and the use amount of the washing liquid is 200 to 1,000µl.

A seventh objective of the present invention is to provide use of a functionalized biological multilayer porous membrane immune carrier in the preparation of a product for blood group detection.

An eighth objective of the present invention is to provide use of a functionalized biological multilayer porous membrane immune carrier in the preparation of a product for detecting and analyzing antibodies including, but not limited to, a pathogenic microbial cell antigen antibody, a bacterial cell antigen antibody or a tumor cell antigen antibody.

In the present invention, a preparation principle of the functionalized biological multilayer porous membrane immune carrier is that: a Fab fragment of the specific anti-biological multilayer porous membrane antibody is bound to the biological multilayer porous membrane, while a Fc fragment is free outwards; and one Fab fragment of the cross-linked antibody can be bound to the free Fc fragment of the specific anti-biological multilayer porous membrane antibody, and the other Fab fragment of the cross-linked antibody can be bound to the specific antibody which belongs to the same species with the specific anti-biological multilayer porous membrane antibody so as to form the functionalized biological multilayer porous membrane immune carrier.

In the present invention, a preparation principle of the biological multilayer porous membrane immune carrier for blood group compatibility detection is that: firstly, the functionalized biological multilayer porous membrane immune carrier is prepared by the method for preparing the functionalized biological multilayer porous membrane immune carrier, as provided by the present invention, and then coupling or an antigen-antibody specific binding reaction is carried out on the cross-linked antibody bound onto the functionalized biological multilayer porous membrane immune carrier and the RBC blood group antibody or the sensitized RBC blood group antigen to prepared the biological multilayer porous membrane immune carrier for blood group compatibility detection, which can be used for blood group detection.

According to the biological multilayer porous membrane immune carrier for blood group compatibility detection, as provided by the present invention, rapid and accurate detection on RBC ABO, Rh, MNS, Kell, Kidd and other blood group system types can be implemented, RBC blood group compatibility detection of forward typing, reverse typing, antibody screening, antibody identification and so on can be simultaneously carried out, and item selection can also be carried out according to Point-of-Care Testing (POCT) demands so as to adapt to various blood group detection requirements and other detection requirements of hospitals, families, field emergencies and the like.

According to the present invention, a hydrophilic biological multilayer porous membrane with the biological activity is prepared from the thick cotton cloth, wool fabric or silkworm cocoons (natural silk fabric) or plant fiber fabric through utilizing immunogenicity and multi-layer porosity of the biological multilayer porous membrane and utilizing biological technologies of the antigen-antibody specific affinity binding interaction and the like; and the hydrophilic biological multilayer porous membrane is bound to the cross-linked antibody after being coupled with the biological multilayer porous membrane specific antibody to prepare the functionalized biological multilayer porous membrane immune carrier, after the functionalized biological multilayer porous membrane immune carrier is coupled with the RBC blood group antibody or the sensitized RBC blood group antigen, the biological multilayer porous membrane immune carrier for blood group compatibility detection is prepared, and the biological multilayer porous membrane immune carrier for blood group compatibility detection, the isolating membrane, the absorbent cotton and the plastic outer shell are assembled sequentially to form the blood group detection card for blood group compatibility detection. If RBCs react with the antibody on the carrier, no matter whether the RBCs are agglutinated, the RBCs can be captured by the antibody on the carrier, and when the membrane shows red after being washed, the positive result is obtained. On the contrary, if the specific antigen-antibody reaction is not performed between the RBCs and the antibody on the carrier, in the process of washing with the washing liquid, the RBCs penetrate through the biological porous membrane and adsorbed away by the absorbent cotton, the membrane shows the natural color, and the negative result is obtained so as to implement rapid blood group forward typing detection. For other blood group compatibility detection, a to-be-detected blood plasma or serum is added into a reaction hole, a blood group antibody in the blood sample reacts with the sensitized RBC blood group antigen coupled to the biological multilayer porous membrane, reverse typing cells or screening cells or panel cells are added, after rinsing is carried out with the washing liquid, the RBCs subjected to the antigen-antibody reaction are captured and fixed on the biological porous membrane and cannot be rinsed away, the biological multilayer porous membrane shows red, the RBCs which are not subjected to the antigen-antibody reaction are rinsed through rinsing fluid and adsorbed away by the absorbent cotton through the porous membrane, and the biological multilayer porous membrane shows the natural color so as to implement reverse typing detection, antibody screening and antibody identification.

Compared with the prior art, the present invention has the beneficial effects that:
(1) The biological multilayer porous membrane adopted by the present invention is from natural materials such as the thick cotton cloth, the wool fabric, the silkworm cocoons (the natural silk fabric), the plant fiber fabric and the like, and is coupled with the corresponding biological multilayer porous membrane antibody and then bound to the cross-linked antibody by utilizing immunogenicity of those natural materials to prepare the functionalized biological multilayer porous membrane immune carrier, and with respect to disordered and nonspecific binding on the plastic surface of a 96-well plate in a conventional Enzyme Linked Immunosorbent Assay (ELISA) experiment, the specific biological multilayer porous membrane carrier is more firm in surface antigen-antibody specific binding, more definite in site and better in specificity.
(2) The biological multilayer porous membrane adopted by the present invention is a multilayer membrane, and thus, the surface area for binding the biological multilayer porous membrane to the RBC blood group antigen or the sensitized RBC blood group antibody is large and much larger than the microporous surface area of the 96-well plate in the conventional ELISA experiment, and then the reaction sensitivity is enhanced.
(3) The biological multilayer porous membrane immune carrier for blood group compatibility detection, which is provided by the present invention, can be specifically bound to the blood group antigen or the blood group antibody in the to-be-detected blood group sample, is high in adsorption capacity and firm in adsorption, shortens the blood group detection time, and improves sensitivity of blood group detection.
(4) According to the blood group detection card provided by the present invention, a solid-phase method is combined with an agglutination method, blood group compatibility detection is implemented, and the porosity characteristic of the multilayer porous membrane can achieve effects of directly rinsing away the RBCs which do not react, simultaneously capturing the single RBCs subjected to the positive reaction as more as possible and entrapping agglutination blocks generated by an agglutination reaction, so that detection is more sensitive, specific and accurate.
(5) According to the blood group detection card provided by the present invention, natural proteins have the protecting effect on biological molecules, and thus, the most important bioactive components in the detection card provided by the present invention are all protected, the antigen antibody coupled to the biological multilayer porous membrane carrier has the preservation temperature of 4 to 50°C, stable antigen activity and antibody titer can be kept, and the detection card is high in stability, is limited a little by the preservation temperature, can be preserved at the normal temperature, is limited a little by outdoor conditions, and is more suitable for use in severe environments.
(6) In some preferred solutions of the present invention, matrices of the isolating membrane, a water absorbent pad, the plastic outer shell and the plastic inner shell included in the blood group detection card structure are all common consumables, which are easy to store, low in cost and high in availability.
(7) In some preferred solutions of the present invention, the adopted isolating membrane includes, but is not limited to, the filter paper, the cellulose fibers, the glass fiber mat, or the fiber mat made of the porous high polymer material or test paper, and the performance and the porosity of the high polymer material of the isolating membrane enables the RBCs to be effectively separated and prevent reverse osmosis, and enables the detection background to be clear so as to accurately and rapidly carry out blood group result interpretation.
(8) In some preferred solutions of the present invention, the adopted water absorbent pad is the cotton pulp mat, the filter paper or other dense water absorbent materials, and can effectively absorb redundant liquid, which is convenient to immediately observe the reaction result, the blood group detection time is shortened, blood group judgment is carried out in one minute, and POCT blood group detection is implemented.
(9) The blood group detection card provided by the present invention adopts a vertical percolation method, so that time required for blood chromatography and washing can be greatly reduced, the operation steps are simplified, and the detection time is shortened.
(10) When the blood group detection card provided by the present invention is used for carrying out detection and forward typing on the to-be-detected blood group sample, the required amount of the to-be-detected blood group sample is 5 to 50µl of human fresh whole blood, the blood volume required for detection is small, and the amount of the sample is saved.
(11) In some preferred solutions of the present invention, the detection hole in the solid-phase plastic carrier adopted by the provided blood group detection card includes, but is not limited to, two holes, three holes or multiple holes, RBC ABO, Rh, Kell, Kidd and other blood group systems which have been discovered and are admitted by the International Society of Blood Transfusion can be detected as required, and reverse typing, antibody screening and antibody identification can be implemented.
(12) According to the detection method provided by the present invention, result determination can adopt naked eyes to carry out manual interpretation, and meanwhile, can also combine conventional spectrum detection to carry out automatic judgment. When manual interpretation is carried out, the difference between the positive result and the negative result is whether there is red, the color difference is obvious, and judgment can be rapidly and accurately carried out. When conventional spectrum detection is combined, batch detection of the blood groups can be implemented so as to satisfy the blood group detection requirement for a great amount of samples.
(13) The functionalized biological multilayer porous membrane immune carrier provided by the present invention not only is used for blood group detection, but also can select the corresponding specific capture antibody as required to perform immunoreactions or continue to carry out immune connection and binding for immunological experiment detection and analysis on antigen antibodies including, but not limited to, the pathogenic microbial cell antigen antibody, the bacterial cell antigen antibody, the tumor cell antigen antibody and various cell antigen antibodies.

The above description is merely an overview of the technical solutions of the present invention, and in order to understand the technical means of the present invention more clearly and enable the present invention to be implemented in accordance with the contents of the description, illustration will be carried out in detail below by some embodiments. The specific implementation modes of the present invention are given out in detail by the following embodiments.

### BRIEF DESCRIPTION OF THE DRAWINGS

The accompanying drawings described herein are used for providing further understanding on the present invention and constitute one part of the present application, and schematic embodiments of the present invention and description thereof are used for explaining the present invention, but do not constitute improper limitation to the present invention. In the accompanying drawings:
Figure 1 is a schematic diagram of ABO forward typing and RhD detection in accordance with the present invention;
Figure 2 is a schematic diagram of ABO reverse typing detection in accordance with the present invention;
Figure 3 is a flow chart of a process for preparing a functionalized biological multilayer porous membrane immune carrier in accordance with the present invention;
Figure 4 is a flow chart of a process for preparing a multilayer porous membrane immune carrier for blood group compatibility detection in accordance with the present invention;
Figure 5 is a structural exploded view of a blood group detection card in accordance with the present invention; and
Figure 6 is a result graph of ABO forward typing detection in one embodiment of the present invention.

Wherein, in the drawings: 1-clamping groove outer shell; 2-clamping groove inner shell; 3-first detection hole; 4-second detection hole; 5-third detection hole; and 6-groove.

### DETAILED DESCRIPTION

In order to more sufficiently understand the present invention described herein, the following embodiments are elaborated.

Firstly, some terms used herein are explained.

"Sensitized RBCs" mean that an incomplete antibody or an autoantibody is bound to a RBC surface antigen or attached to a RBC membrane to sensitize the RBCs.

"RBC panel cells" refer to a group of multiple parts of RBCs carrying most of clinically significant blood group antigens and used for RBC blood group identification.

"RBC membrane antibody of a non-blood group antibody" refers to a RBC membrane antibody, but is not an antibody against a RBC blood group system.

In order to more sufficiently understand the present invention described herein, the following embodiments are elaborated.

### Embodiment 1

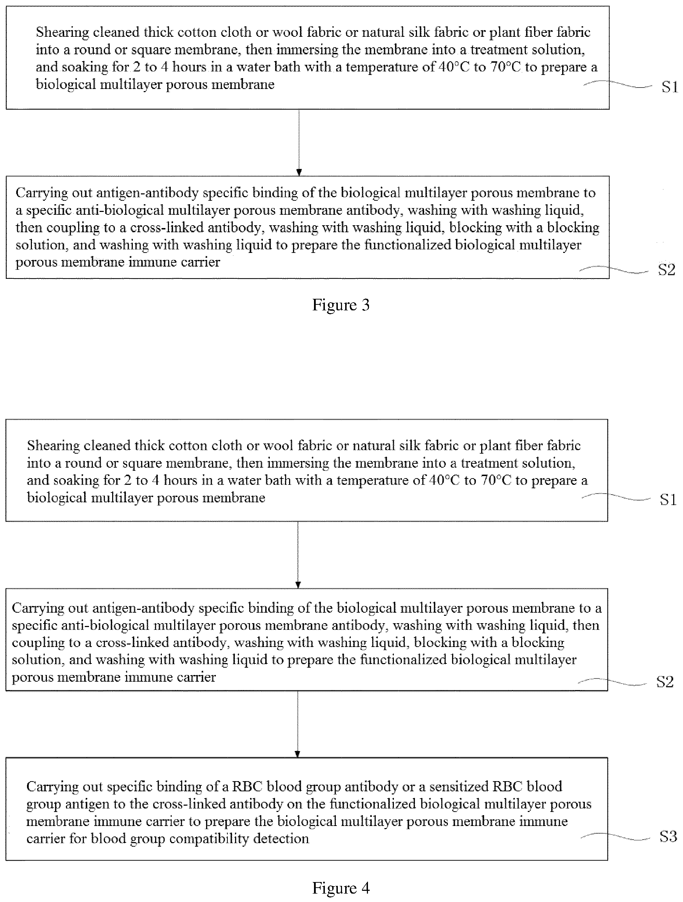

As shown in Figure 3, preparation of a functionalized biological multilayer porous membrane immune carrier includes the following steps of:

### 1) Preparation of a biological multilayer porous membrane

Firstly, silkworm cocoons (a natural silk fabric) are sheared from a long axis direction, and silkworm chrysalis, impurity silk and other impurities are removed. The obtained product is washed with pure water twice, placed into water with the temperature of 100°C to be soaked for 15min, and air-dried at room temperature after being taken out. Then the obtained product is punched by a hole puncher to form round biological multilayer porous membranes with a specification of 5 to 9mm. A treatment solution is added to soak silkworm pieces, and incubation is carried out in a water bath with the temperature of 50°C for 2 hours. Vacuum bubble removal is carried out once by a vacuum dryer, all the round biological multilayer porous membranes are sunk to the bottom of the treatment solution, incubation is continued to be carried out in the water bath with the temperature of 50°C for 2 hours, and the obtained product is washed twice with washing liquid to prepare the required biological multilayer porous membrane.

### 2) Preparation of the functionalized biological multilayer porous membrane immune carrier

An anti-silk protein antibody (the use amount of the antibody is about 200µl per piece) with a final concentration of 20µg/ml is added into the washed biological multilayer porous membrane in the step 1), an oscillation reaction is carried out at room temperature for 2 hours, and the obtained product is reserved overnight at the temperature of 4°C. Washing is carried out with washing liquid for 3 times, detection and identification are carried out by HRP-anti-mouse Ig(G+A+M) capable of being purchased commercially, after a reaction is performed for 1 hour at the temperature of 37°C, washing is carried out with washing liquid for 3 times, 200µl per piece of precipitating substrate is added, color development is carried out for 15min, and then the membrane completely shows dark purple. A goat anti-mouse IgG cross-linked antibody is added into the biological multilayer porous membrane coupled with the anti-silk protein antibody, the final concentration is 20µg/ml, the oscillation reaction is performed at room temperature for 2 hours, and the obtained product is reserved overnight at the temperature of 4°C. Washing is carried out with washing liquid for 3 times, detection and identification are carried out by HRP-anti-GST, after the reaction is performed for 1 hour at the temperature of 37°C, blocking is carried out with a blocking solution for 2 hours at the temperature of 37°C, washing is carried out with washing liquid for 3 times, and then the membrane completely shows dark purple so as to prepare the functionalized biological multilayer porous membrane immune carrier, wherein the membrane needs to be completely soaked in the coupling and blocking process.

### Embodiment 2

As shown in Figure 4, preparation of a biological multilayer porous membrane immune carrier for human ABO, Rh(D) blood group forward typing detection includes the following steps of:
1) Preparation of a functionalized biological multilayer porous membrane immune carrier according to the method in Embodiment 1; and
2) Coupling of the functionalized biological multilayer porous membrane immune carrier and a RBC blood group antibody

Monoclonal antibody-A and antibody-B with the titer of 128, which can be commercially purchased, are respectively added into the functionalized biological multilayer porous membrane immune carrier obtained in the step 1), and the mixture is reserved overnight at the temperature of 4°C. Air-drying is carried out at room temperature of 20 to 25°C, and the air-dried product is stored at the temperature of 4°C for later use so as to prepare the biological multilayer porous membrane immune carrier for human ABO blood group detection.

Monoclonal anti-human IgG is added into the other part of biological multilayer porous membrane immune carrier, the final concentration is 20µg/ml, after the mixture is reserved overnight at the temperature of 4°C, washing is carried out with washing liquid for 3 times, anti-D with the titer of 512 is added, and the obtained product is reserved overnight at the temperature of 4°C. Air-drying is carried out at room temperature of 20 to 25°C, and the air-dried product is stored at the temperature of 4°C for later use so as to prepare the biological multilayer porous membrane immune carrier for human Rh(D) blood group detection.

### Embodiment 3

Preparation of a biological multilayer porous membrane immune carrier for human ABO blood group reverse typing detection includes the following steps of:
1) Preparation of a functionalized biological multilayer porous membrane immune carrier according to the method in Embodiment 1;
2) Preparation of sensitized RBC blood group antigens
   Fresh group-A, group-B and group-O RBCs are taken and are respectively prepared into 50% RBC suspension by normal saline, monoclonal anti-A, anti-B and anti-H (the titer is 8) with the same volume are respectively added, a reaction is performed at room temperature for 5 minutes, at the moment, the RBCs will be agglutinated in a fine sand shape (the intensity of agglutination is 1+), a hypotonic solution (0.1% to 0.7% NaCl solution) of which the volume is 5 times of the volume of the agglutinated RBCs, and after standing is carried out for 1 minute at room temperature, centrifugation is carried out at the revolution speed of 12,000rpm for 5 minutes. Supernate is discarded, and the sensitized RBC blood group antigens are collected and are suspended by normal saline to the original volume.
3) Preparation of the biological multilayer porous membrane immune carrier for human ABO blood group reverse typing detection by antibody-antigen specific binding of the functionalized biological multilayer porous membrane immune carrier and the sensitized RBC blood group antigen

The sensitized group-A, group-B and group-O RBC antigens prepared in the step 2) are added into the functionalized biological multilayer porous membrane immune carrier obtained in the step 1), and a reaction is performed overnight at the temperature of 4°C. The obtained product is taken out, air-dried at the temperature of 20 to 25°C and stored at the temperature of 4°C for later use so as to prepare the biological multilayer porous membrane immune carrier for human ABO blood group reverse typing detection.

### Embodiment 4

Preparation of a biological multilayer porous membrane immune carrier for RBC blood group antibody screening includes the following steps:
1) Preparation of a functionalized biological multilayer porous membrane immune carrier according to the method in Embodiment 1;
2) Preparation of irregular antibody screening RBC antigens
   Irregular antibody screening RBCs I, II and III respectively from three people are taken, and are respectively prepared into 50% RBC suspension by normal saline, monoclonal anti-H (the titer is 8) with the same volume is respectively added, a reaction is performed at room temperature for 5 minutes, at the moment, the RBCs will be agglutinated in a fine sand shape (the intensity of agglutination is 1+), a hypotonic solution (0.1% to 0.7% NaCl solution) of which the volume is 5 times of the volume of the agglutinated RBCs, and after standing is carried out for 1 minute at room temperature, centrifugation is carried out at the revolution speed of 12,000rpm for 5 minutes. Supernate is discarded, and sensitized irregular antibody screening RBC antigens I, II and III are collected. The sensitized irregular antibody screening RBC antigens I, II and III are suspended by normal saline to the original volume.
3) Antibody-antibody specific binding of the functionalized biological multilayer porous membrane immune carrier and the irregular antibody screening RBC antigens

The irregular antibody screening RBC antigens I, II and III prepared in the step 2) are added into the functionalized biological multilayer porous membrane immune carrier obtained in the step 1), and a reaction is performed overnight at the temperature of 4°C. The obtained product is taken out, air-dried at the temperature of 20 to 25°C and stored at the temperature of 4°C for later use so as to prepare the biological multilayer porous membrane immune carrier for RBC blood group antibody screening.

### Embodiment 5

Preparation of a biological multilayer porous membrane immune carrier for RBC blood group antibody identification includes the following steps of:
1) Preparation of a functionalized biological multilayer porous membrane immune carrier according to the method in Embodiment 1;
2) Preparation of sensitized RBC panel cell antigens
   12 groups of RBC panel cells are taken and respectively prepared into 50% RBC suspension by normal saline, monoclonal anti-H (the titer is 8) with the same volume is respectively added, a reaction is performed at room temperature for 5 minutes, at the moment, the RBCs will be agglutinated in a fine sand shape (the intensity of agglutination is 1+), a hypotonic solution (0.1% to 0.7% NaCl solution) of which the volume is 5 times of the volume of the agglutinated RBCs, and after standing is carried out for 1 minute at room temperature, centrifugation is carried out at the revolution speed of 12,000rpm for 5 minutes. Supernate is discarded, and 12 groups of sensitized RBC panel cell antigens are collected and are suspended by normal saline to the original volume.
3) Antigen-antibody specific binding of the functionalized biological multilayer porous membrane immune carrier and the sensitized RBC panel cell antigens.

12 groups of RBC panel cell antigens prepared in the step 2) are respectively added into 12 parts of the functionalized biological multilayer porous membrane immune carrier prepared in the step 1), and a reaction is performed overnight at the temperature of 4°C. The obtained product is taken out, air-dried at the temperature of 20 to 25°C and stored at the temperature of 4°C for later use so as to prepare the biological multilayer porous membrane immune carrier for RBC blood group antibody identification.

It should be explained that in Embodiments 1-5, the specific biological multilayer porous membrane antibody is diluted by a diluent to have a final concentration of 1 to 100µg/ml; and the cross-linked antibody is diluted by the diluent to have a final concentration of 1 to 100µg/ml, wherein the diluent is a phosphate buffer solution with a concentration of 0.01mol/L and a pH of 7.0 to 7.2.

The treatment solution is a phosphate buffer solution with a concentration of 0.01mol/L and a pH of 7.0 to 7.2, which contains 1% bovine serum albumin; the washing liquid is a phosphate buffer solution with a concentration of 0.01mol/L and a pH of 7.0 to 7.2; and the blocking solution is 5% skim milk powder prepared by a phosphate buffer solution with a concentration of 0.01mol/L and a pH of 7.0 to 7.2, wherein the unit % represents a mass of a solute in 100ml of solution.

### Embodiment 6

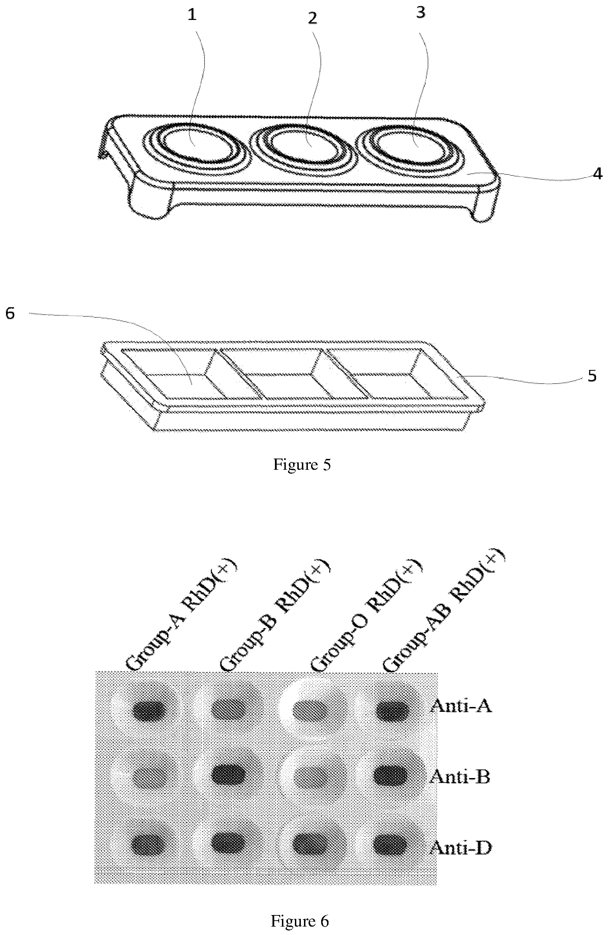

As shown in Figure 5, a blood group detection card includes a solid-phase plastic carrier made of a polyethylene material. The solid-phase plastic carrier includes a clamping groove outer shell 1 and a clamping groove inner shell 2, and the clamping groove outer shell 1 and the clamping groove inner shell 2 are in detachable connection; the clamping groove outer shell 1 of the blood group detection card is also provided with a first detection hole 3, a second detection hole 4 and a third detection hole 5; the biological multilayer porous membrane immune carrier for blood group compatibility detection, which is prepared in any one of Embodiment 2 to Embodiment 5, is placed at the bottoms of the first detection hole 3, the second detection hole 4 and the third detection hole 5; the clamping groove inner shell 2 is provided with three grooves 6 corresponding to the first detection hole 3, the second detection hole 4 and the third detection hole 5, absorbent cotton is placed at the bottoms of the grooves 6, and the absorbent cotton is a cotton pulp mat; and an isolating membrane is placed on the absorbent cotton, and the isolating membrane is a glass fiber mat.

It should be understood that the number of the detection holes is not merely limited to three and is specifically set according to a detection object and detection conditions of the detection card, and the detection hole may include two holes, three holes and multiple holes.

### Embodiment 7

ABO forward typing and RhD blood group detection and detection result
The blood group detection card provided by Embodiment 6 is adopted, the number of the detection holes is 3, and the biological multilayer porous membrane immune carrier coupled with anti-A, anti-B and anti-D prepared in Embodiment 2 is respectively arranged at the bottoms of the first detection hole 3, the second detection hole 4 and the third detection hole 5.

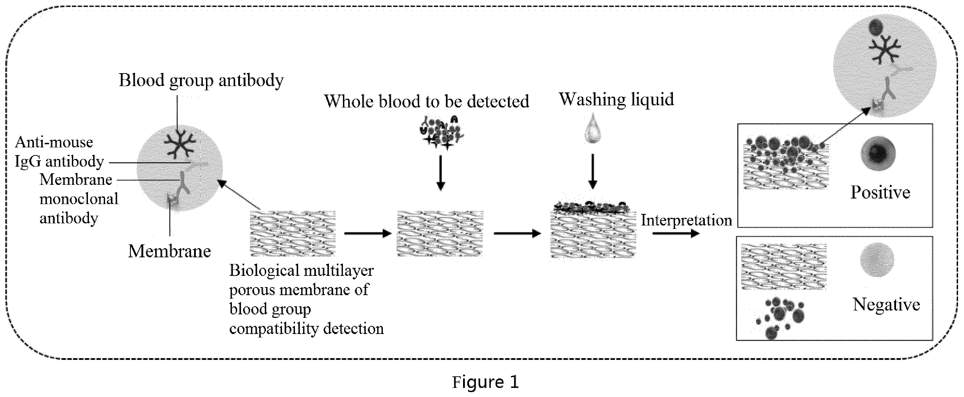

Fresh whole blood is taken, 10µl of whole blood is respectively dropwise added into the first detection holes 3, 2, 3, and standing is carried out at room temperature for 1 minute. Three drops of rinsing fluid (a buffer solution containing 0.05% Tween-20, of which the pH value is 7.0) are respectively dropwise added into each hole, then the RBC distribution condition is observed and a detection result is determined. A detection principle is as shown in Figure 1.

As shown in Figure 6, Figure 6 shows a display result of the detection card according to this embodiment. RBCs subjected to an antigen-antibody reaction are captured, fixed and blocked on the biological porous membrane and cannot be rinsed away, the RBCs are enriched in the detection hole, the detection hole shows red, and a positive result is obtained; and on the contrary, the RBCs not subjected to the antigen-antibody reaction is rinsed by the rinsing fluid and is absorbed by the absorbent cotton through the porous membrane, no RBC is enriched in the detection hole, the detection hole shows the natural color, and then a negative result is obtained, wherein if anti-A is bound to the biological multilayer porous membrane immune carrier for blood group compatibility detection at the bottom of the detection hole, the natural color of the detection hole is blue; if anti-B is bound, the natural color of the detection hole is yellow; and if anti-D is bound, the natural color of the detection hole is colourless. Finally, a result is identified and determined according to the detection hole with the positive result.

Figure 6 shows a color development condition of a color development hole. Figure 6 is not a colored drawing and thus is not obvious in color observation. A color display result of this embodiment can further refer to Table 1.

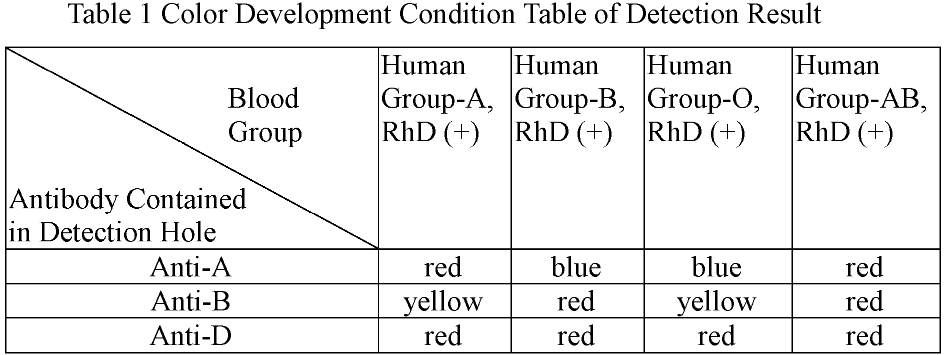

### Embodiment 8

### ABO reverse typing detection

The blood group detection card provided by Embodiment 6 is adopted, the number of the detection holes is 3, and the biological multilayer porous membrane immune carrier specifically bound to a sensitized RBC group-A antigen, a sensitized RBC group-B antigen and a sensitized RBC H antigen prepared in Embodiment 3 is respectively arranged at the bottoms of the first detection hole 3, the second detection hole 4 and the third detection hole 5.

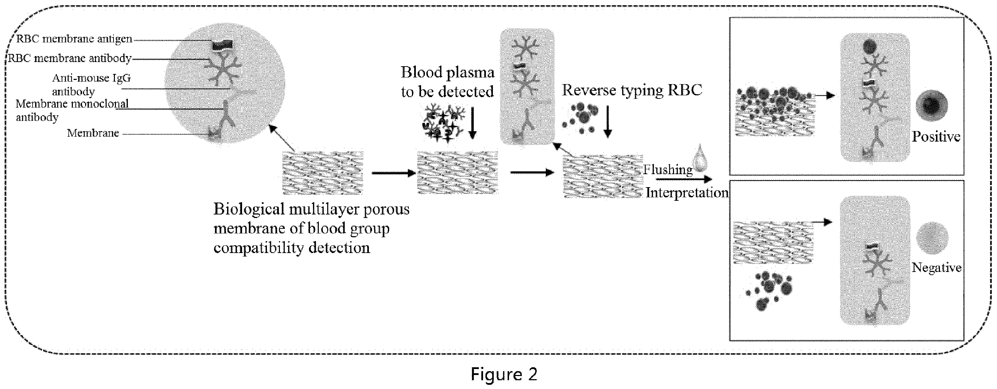

A fresh to-be-detected sample (blood plasma or serum) is taken, one drop of the sample is respectively dropwise added into the first detection holes 3, 2, 3, and standing is carried out at room temperature for 1 minute. One drop of ABO reverse typing RBCs is respectively added into the corresponding holes, and standing is carried out at room temperature for 1 minute. Then three drops of rinsing fluid (a buffer solution containing 0.05% Tween-20, of which the pH value is 7.0) are respectively dropwise added into each of the corresponding holes, the RBC distribution condition is observed and a detection result is determined. A detection principle is as shown in Figure 2.

RBCs subjected to an antigen-antibody reaction are captured and fixed on the biological porous membrane and cannot be rinsed away, the RBCs are enriched in the detection hole, the detection hole shows red, and a positive result is obtained; and on the contrary, the RBCs not subjected to the antigen-antibody reaction is rinsed by the rinsing fluid and is absorbed by the absorbent cotton through the porous membrane, no RBC is enriched in the detection hole, the detection hole shows the natural color of the membrane, and then a negative result is obtained. Finally, an ABO reverse typing result is identified and determined according to the detection hole with the positive result.

### Embodiment 9

### Irregular antibody screening

The blood group detection card provided by Embodiment 6 is adopted, the number of the detection holes is 3, and the biological multilayer porous membrane immune carriers for RBC blood group antibody identification, which are respectively prepared by irregular antibody screening RBCs I, II and III of three people in Embodiment 4 are respectively arranged at the bottoms of the first detection hole 3, the second detection hole 4 and the third detection hole 5.

A fresh to-be-detected sample (blood plasma or serum) is taken, one drop of the sample is respectively dropwise added into the detection holes, and standing is carried out at room temperature for 1 minute. One drop of irregular antibody screening RBCs I, one drop of irregular antibody screening RBCs II and one drop of irregular antibody screening RBCs III are respectively added into the corresponding holes, and standing is carried out at room temperature for 1 minute. Then three drops of rinsing fluid (a buffer solution containing 0.05% Tween-20, of which the pH value is 7.0) are respectively dropwise added into each of the corresponding holes, the RBC distribution condition is observed and a detection result is determined. A detection principle is as shown in Figure 2.

RBCs subjected to an antigen-antibody reaction are captured and fixed on the biological porous membrane and cannot be rinsed away, the RBCs are enriched in the detection hole, the detection hole shows red, and a positive result is obtained; and on the contrary, the RBCs not subjected to the antigen-antibody reaction is rinsed by the rinsing fluid and is absorbed by the absorbent cotton through the porous membrane, no RBC is enriched in the detection hole, the detection hole shows the natural color, and then a negative result is obtained. Finally, an antibody screening result is identified and determined according to the detection hole with the positive result.

### Embodiment 10

### RBC antibody identification

The blood group detection card provided by Embodiment 6 is adopted, the number of the detection holes is 12, and the biological multilayer porous membrane immune carriers for RBC blood group antibody identification, which are respectively prepared by 12 groups of sensitized RBC panel cell antigens in Embodiment 5 are respectively arranged at the bottoms of the first detection holes 3-12.

A fresh to-be-detected sample (blood plasma or serum) is taken, one drop of the sample is respectively dropwise added into 12 detection holes, and standing is carried out at room temperature for 1 minute. One drop of RBC panel cells is respectively added into the corresponding holes, and standing is carried out at room temperature for 1 minute. Then three drops of rinsing fluid are respectively dropwise added into each of the corresponding holes, the RBC distribution condition is observed and a detection result is determined. A detection principle is as shown in Figure 2.

RBCs subjected to an antigen-antibody reaction are captured and fixed on the biological porous membrane and cannot be rinsed away, the RBCs are enriched in the detection hole, the detection hole shows red, and a positive result is obtained; and on the contrary, the RBCs not subjected to the antigen-antibody reaction is rinsed by the rinsing fluid and is absorbed by the absorbent cotton through the porous membrane, no RBC is enriched in the detection hole, the detection hole shows the natural color, and then a negative result is obtained. Finally, an antibody identification result is identified and determined according to the detection hole with the positive result.

The detection method disclosed by the present invention is beneficial for promoting clinical RBC blood group compatibility detection including RBC forward typing, reverse typing, antibody screening, antibody identification and the like, rapid and accurate detection on RBC ABO, Rh, MNS, Kell, Kidd and other blood group system types can be implemented, and selection of different detection items can also be carried out according to POCT demands so as to adapt to various blood group detection cases of hospitals, families, field emergencies and the like.

The detection card provided by the present invention needed to be preserved or used at 4°C to 50°C, and the defect of strict requirements of an existing blood group detection product for the preservation or detection temperature is overcome.

## Claims

1. A functionalized biological multilayer porous membrane immune carrier, comprising:
a biological multilayer porous membrane;
a specific anti-biological multilayer porous membrane antibody, the specific anti-biological multilayer porous membrane antibody being attached to the surface of the biological multilayer porous membrane through antigen-antibody specific binding with the biological multilayer porous membrane; and
a cross-linked antibody, the cross-linked antibody being coupled and bound with the specific anti-biological multilayer porous membrane antibody,
wherein one Fab terminal of the cross-linked antibody is capable of being coupled with the specific anti-biological multilayer porous membrane antibody, and the other Fab terminal is capable of being bound to a specific antibody.

2. The functionalized biological multilayer porous membrane immune carrier of claim 1, **characterized in that**, the specific anti-biological multilayer porous membrane antibody comprises a murine monoclonal antibody, a goat derived polyclonal antibody, a rabbit derived monoclonal antibody/polyclonal antibody or a chicken derived polyclonal antibody; the cross-linked antibody comprises an anti-mouse polyclonal antibody, an anti-goat polyclonal antibody, an anti-rabbit polyclonal antibody or an anti-chicken polyclonal antibody; and the specific antibody which is capable of being bound to the other Fab terminal of the cross-linked antibody is a murine monoclonal antibody, a goat derived polyclonal antibody, a rabbit derived monoclonal/polyclonal antibody or a chicken derived polyclonal antibody.

3. A method for preparing a functionalized biological multilayer porous membrane immune carrier, comprising the following steps of:
S1: shearing cleaned thick cotton cloth or wool fabric or natural silk fabric or plant fiber fabric into a round or square membrane, then immersing the membrane into a treatment solution, and soaking for 2 to 4 hours in a water bath with a temperature of 40°C to 70°C to prepare a biological multilayer porous membrane; and
S2: carrying out antigen-antibody specific binding of the biological multilayer porous membrane to a specific anti-biological multilayer porous membrane antibody, washing with washing liquid, then coupling to a cross-linked antibody, washing with washing liquid, blocking with a blocking solution, and washing with washing liquid to prepare the functionalized biological multilayer porous membrane immune carrier of claim 1,
wherein the specific anti-biological multilayer porous membrane antibody is an antibody capable of undergoing an antigen-antibody specific reaction with the biological multilayer porous membrane; and
one Fab terminal of the cross-linked antibody is capable of being coupled with the specific anti-biological multilayer porous membrane antibody, and the other Fab terminal is capable of being bound to a specific antibody.

4. A biological multilayer porous membrane immune carrier for blood group compatibility detection, comprising:
the functionalized biological multilayer porous membrane immune carrier of claim 1 or 2; and
a red blood cell blood group antibody or a sensitized red blood cell blood group antigen, attached to the functionalized biological multilayer porous membrane immune carrier through specific binding with the cross-linked antibody on the functionalized biological multilayer porous membrane immune carrier,
wherein the sensitized red blood cell blood group antigen is a red blood cell blood group antigen specifically bound to a red blood cell membrane antibody of a non-blood group antibody.

5. The biological multilayer porous membrane immune carrier for blood group compatibility detection of claim 4, **characterized in that**, the red blood cell blood group antibody comprises blood group antibodies of IgM type, IgG type or IgM-IgG mixed type for ABO, Rh, MNS, Kell and Kidd blood group systems, and the red blood cell blood group antibody has a titer of greater than 1:32.

6. The biological multilayer porous membrane immune carrier for blood group compatibility detection of claim 4, **characterized in that**, the sensitized red blood cell blood group antigen comprises a sensitized A-type red blood cell antigen, a sensitized B-type red blood cell antigen, a sensitized irregular antibody screening red blood cell antigen or a sensitized red blood cell panel cell antigen.

7. A method for preparing a biological multilayer porous membrane immune carrier for blood group compatibility detection, comprising the following steps of:
S1: shearing cleaned thick cotton cloth or wool fabric or natural silk fabric or plant fiber fabric into a round or square membrane, then immersing the membrane into a treatment solution, and soaking for 2 to 4 hours in a water bath with a temperature of 40°C to 70°C to prepare a biological multilayer porous membrane;
S2: carrying out antigen-antibody specific binding of the biological multilayer porous membrane to a specific anti-biological multilayer porous membrane antibody, washing with washing liquid, then coupling to a cross-linked antibody, washing with washing liquid, blocking with a blocking solution, and washing with washing liquid to prepare the functionalized biological multilayer porous membrane immune carrier of claim 1; and
S3: carrying out specific binding of a red blood cell blood group antibody or a sensitized red blood cell blood group antigen to the cross-linked antibody on the functionalized biological multilayer porous membrane immune carrier to prepare the biological multilayer porous membrane immune carrier for blood group compatibility detection of claim 4,
wherein the specific anti-biological multilayer porous membrane antibody is an antibody capable of undergoing an antigen-antibody specific reaction with the biological multilayer porous membrane;
one Fab terminal of the cross-linked antibody is capable of being coupled with the specific anti-biological multilayer porous membrane antibody, and the other Fab terminal is capable of being bound to a specific antibody, and the specific antibody is a red blood cell blood group antibody or a sensitized red blood cell blood group antigen capable of being bound to the Fab terminal of the cross-linked antibody; and
the sensitized red blood cell blood group antigen is a red blood cell blood group antigen specifically bound to a red blood cell membrane antibody of a non-blood group antibody.

8. The method for preparing the biological multilayer porous membrane immune carrier for blood group compatibility detection of claim 7, further comprising a step of preparing the sensitized red blood cell blood group antigen as follows: reacting the red blood cell membrane antibody of the non-blood group antibody with fresh red blood cells, lysing the red blood cells by a hypotonic solution when the intensity of agglutination reaches 1+, centrifuging and collecting to obtain the sensitized red blood cell antigen.

9. A blood group detection card, comprising:
a solid-phase plastic carrier, comprising a clamping groove outer shell and a clamping groove inner shell, the clamping groove outer shell and the clamping groove inner shell being in detachable connection;
the biological multilayer porous membrane immune carrier for blood group compatibility detection of any one of claims 4-6, positioned at the bottom of the clamping groove outer shell of the solid-phase plastic carrier;
an absorbent cotton, positioned at the bottom of the clamping groove inner shell of the solid-phase plastic carrier; and
an isolating membrane, arranged above the water absorbent cotton.

10. The blood group detection card of claim 9, **characterized in that**, the solid-phase plastic carrier is made of a material selected from polyethylene, polystyrene and styrene-butadiene-acrylonitrile; the solid-phase plastic carrier further comprises a detection hole, comprising two holes, three holes or multiple holes; the isolating membrane comprises a filter paper, cellulose fibers, a glass fiber mat, a fiber mat made of a porous high polymer material or a test paper; and the absorbent cotton comprises a cotton pulp mat and a filter paper.

11. A method for detecting a blood group, comprising the following steps of:
adding a to-be-detected blood group sample into the detection hole of the blood group detection card of any one of claims 9-10, and allowing the to-be-detected blood group sample to stand for at least 1 minute; and rinsing with washing liquid, observing the color development of the detection hole, wherein red color development indicates a positive result, and a natural color development indicates a negative result,
wherein a positive result is observed if the to-be-detected blood group sample undergoes an antigen-antibody specific reaction with the biological multilayer porous membrane immune carrier for blood group compatibility detection in the blood group detection card; and otherwise, the result is negative.

12. Use of the functionalized biological multilayer porous membrane immune carrier of claim 1 or 2 in the preparation of a product for blood group compatibility detection.

13. Use of the functionalized biological multilayer porous membrane immune carrier of claim 1 or 2 in the preparation of a product for detecting and analyzing a pathogenic microbial cell antigen antibody, a bacterial cell antigen antibody or a tumor cell antigen antibody.

## Patentansprüche

1. Funktionalisierter biologischer mehrschichtiger poröser Membran-Immunträger, umfassend:
eine biologische mehrschichtige poröse Membran;
einen spezifischen anti-biologischen mehrschichtigen porösen Membran-Antikörper, wobei der spezifische anti-biologische mehrschichtige poröse Membran-Antikörper an die Oberfläche der biologischen mehrschichtigen porösen Membran durch eine Antigen-Antikörper-spezifische Bindung mit der biologischen mehrschichtigen porösen Membran befestigt ist; und
einen vernetzten Antikörper, wobei der vernetzte Antikörper mit dem spezifischen anti-biologischen mehrschichtigen porösen Membran-Antikörper gekoppelt und gebunden ist,
wobei ein Fab-Fragment des vernetzten Antikörpers in der Lage ist, mit dem spezifischen anti-biologischen mehrschichtigen porösen Membran-Antikörper gekoppelt zu werden und das andere Fab-Fragment in der Lage ist, an einen spezifischen Antikörper gebunden zu werden.

2. Funktionalisierter biologischer mehrschichtiger poröser Membran-Immunträger nach Anspruch 1, **dadurch gekennzeichnet, dass** der spezifische anti-biologische mehrschichtige poröse Membran-Antikörper einen monoklonalen Maus-Antikörper, einen aus Ziege gewonnenen polyklonalen Antikörper, einen aus Kaninchen gewonnenen monoklonalen Antikörper/polyklonalen Antikörper oder einen aus Huhn gewonnenen polyklonalen Antikörper umfasst; der vernetzte Antikörper einen polyklonalen Anti-Maus-Antikörper, einen polyklonalen Anti-Ziege-Antikörper, einen polyklonalen Anti-Kaninchen-Antikörper oder einen polyklonalen Anti-Huhn-Antikörper umfasst; und der spezifische Antikörper, der in der Lage ist, an das andere Fab-Fragment des vernetzten Antikörpers gebunden zu werden, ein monoklonaler Maus-Antikörper, ein aus Ziege gewonnener polyklonaler Antikörper, ein aus Kaninchen gewonnener monoklonaler/polyklonaler Antikörper oder ein aus Huhn gewonnener polyklonaler Antikörper ist.

3. Verfahren zur Herstellung eines funktionalisierten biologischen mehrschichtigen porösen Membran-Immunträgers, umfassend die folgenden Schritte:
S1: Zuschneiden eines gereinigten dicken Baumwolltuchs oder Wollstoffs oder eines natürlichen Seidenstoffs oder eines Pflanzenfaserstoffs zu einer runden oder quadratischen Membran, anschließendes Eintauchen der Membran in eine Behandlungslösung und Einweichen für 2 bis 4 Stunden in einem Wasserbad mit einer Temperatur von 40 °C bis 70 °C, um eine biologische mehrschichtige poröse Membran herzustellen; und
S2: Durchführen einer Antigen-Antikörper-spezifischen Bindung der biologischen mehrschichtigen porösen Membran an einen spezifischen anti-biologischen mehrschichtigen porösen Membran-Antikörper, Waschen mit Waschflüssigkeit, dann Koppeln an einen vernetzten Antikörper, Waschen mit Waschflüssigkeit, Blockieren mit einer Blockierlösung und Waschen mit Waschflüssigkeit, um den funktionalisierten biologischen mehrschichtigen porösen Membran-Immunträger nach Anspruch 1 herzustellen,
wobei der spezifische anti-biologische mehrschichtige poröse Membran-Antikörper ein Antikörper ist, der in der Lage ist, eine Antigen-Antikörper-spezifische Reaktion mit der biologischen mehrschichtigen porösen Membran einzugehen; und
ein Fab-Fragment des vernetzten Antikörpers in der Lage ist, mit dem spezifischen anti-biologischen mehrschichtigen porösen Membran-Antikörper gekoppelt zu werden und das andere Fab-Fragment in der Lage ist, an einen spezifischen Antikörper gebunden zu werden.

4. Biologischer mehrschichtiger poröser Membran-Immunträger zur Blutgruppenkompatibilitätsdetektion, umfassend:
den funktionalisierten biologischen mehrschichtigen porösen Membran-Immunträger nach Anspruch 1 oder 2; und
einen Erythrozyten-Blutgruppen-Antikörper oder ein sensibilisiertes Erythrozyten-Blutgruppen-Antigen, das an den funktionalisierten biologischen mehrschichtigen porösen Membran-Immunträger durch spezifische Bindung mit dem vernetzten Antikörper auf dem funktionalisierten biologischen mehrschichtigen porösen Membran-Immunträger befestigt ist,
wobei das sensibilisierte Erythrozyten-Blutgruppen-Antigen ein Erythrozyten-Blutgruppen-Antigen ist, das spezifisch an einen Erythrozyten-Membran-Antikörper eines Nicht-Blutgruppen-Antikörpers gebunden ist.

5. Biologischer mehrschichtiger poröser Membran-Immunträger zur Blutgruppenkompatibilitätsdetektion nach Anspruch 4, **dadurch gekennzeichnet, dass** der Erythrozyten-Blutgruppen-Antikörper Blutgruppen-Antikörper vom IgM-Typ, IgG-Typ oder IgM-IgG-Mischtyp für ABO-, Rh-, MNS-, Kell- und Kidd-Blutgruppensysteme umfasst, und der Erythrozyten-Blutgruppen-Antikörper einen Titer von mehr als 1:32 aufweist.

6. Biologischer mehrschichtiger poröser Membran-Immunträger zur Blutgruppenkompatibilitätsdetektion nach Anspruch 4, **dadurch gekennzeichnet, dass** das sensibilisierte Erythrozyten-Blutgruppen-Antigen ein sensibilisiertes Typ-A-Erythrozyten-Antigen, ein sensibilisiertes Typ-B-Erythrozyten-Antigen, ein sensibilisiertes irreguläres Antikörper-Screening-Erythrozyten-Antigen oder ein sensibilisiertes Erythrozyten-Panelzell-Antigen umfasst.

7. Verfahren zur Herstellung eines biologischen mehrschichtigen porösen Membran-Immunträgers zur Blutgruppenkompatibilitätsdetektion, umfassend die folgenden Schritte:
S1: Zuschneiden eines gereinigten dicken Baumwolltuchs oder Wollstoffs oder eines natürlichen Seidenstoffs oder eines Pflanzenfaserstoffs zu einer runden oder quadratischen Membran, anschließendes Eintauchen der Membran in eine Behandlungslösung und Einweichen für 2 bis 4 Stunden in einem Wasserbad mit einer Temperatur von 40 °C bis 70 °C, um eine biologische mehrschichtige poröse Membran herzustellen;
S2: Durchführen einer Antigen-Antikörper-spezifischen Bindung der biologischen mehrschichtigen porösen Membran an einen spezifischen anti-biologischen mehrschichtigen porösen Membran-Antikörper, Waschen mit Waschflüssigkeit, dann Koppeln an einen vernetzten Antikörper, Waschen mit Waschflüssigkeit, Blockieren mit einer Blockierlösung und Waschen mit Waschflüssigkeit, um den funktionalisierten biologischen mehrschichtigen porösen Membran-Immunträger nach Anspruch 1 herzustellen; und
S3: Durchführen einer spezifischen Bindung eines Erythrozyten-Blutgruppen-Antikörpers oder eines sensibilisierten Erythrozyten-Blutgruppen-Antigens an den vernetzten Antikörper auf dem funktionalisierten biologischen mehrschichtigen porösen Membran-Immunträger, um den biologischen mehrschichtigen porösen Membran-Immunträger zur Blutgruppenkompatibilitätsdetektion nach Anspruch 4 herzustellen,
wobei der spezifische anti-biologische mehrschichtige poröse Membran-Antikörper ein Antikörper ist, der in der Lage ist, eine Antigen-Antikörper-spezifische Reaktion mit der biologischen mehrschichtigen porösen Membran einzugehen;
ein Fab-Fragment des vernetzten Antikörpers, das in der Lage ist, mit dem spezifischen anti-biologischen mehrschichtigen porösen Membran-Antikörper gekoppelt zu werden, und das andere Fab-Fragment das in der Lage ist, an einen spezifischen Antikörper gebunden zu werden, und der spezifische Antikörper ein Erythrozyten-Blutgruppen-Antikörper oder ein sensibilisiertes Erythrozyten-Blutgruppen-Antigen ist, das in der Lage ist, an das Fab-Fragment des vernetzten Antikörpers gebunden zu werden; und
das sensibilisierte Erythrozyten-Blutgruppen-Antigen ein Erythrozyten-Blutgruppen-Antigen ist, das spezifisch an einen Erythrozyten-Membran-Antikörper eines Nicht-Blutgruppen-Antikörpers gebunden ist.

8. Verfahren zur Herstellung des biologischen mehrschichtigen porösen Membran-Immunträgers zur Blutgruppenkompatibilitätsdetektion nach Anspruch 7, ferner umfassend einen Schritt der Herstellung des sensibilisierten Erythrozyten-Blutgruppen-Antigens wie folgt: Reagieren des Erythrozyten-Membran-Antikörpers des Nicht-Blutgruppen-Antikörpers mit frischen Erythrozyten, Lysieren der roten Blutkörperchen durch eine hypotonische Lösung, wenn die Intensität der Agglutination 1+ erreicht, Zentrifugieren und Sammeln, um das sensibilisierte Erythrozyten-Antigen zu erhalten.

9. Blutgruppendetektionskarte, umfassend:
einen Festphasenkunststoffträger, umfassend eine Klemmvertiefungsaußenschale und eine Klemmvertiefungsinnenschale, wobei die Klemmvertiefungsaußenschale und die Klemmvertiefungsinnenschale in lösbarer Verbindung stehen;
den biologischen mehrschichtigen porösen Membran-Immunträger zur Blutgruppenkompatibilitätsdetektion nach einem der Ansprüche 4-6, positioniert am Boden der Klemmvertiefungsaußenschale des Festphasenkunststoffträgers;
eine saugfähige Baumwolle, positioniert am Boden der Klemmvertiefungsinnenschale des Festphasenkunststoffträgers; und
eine isolierende Membran, angeordnet oberhalb der wasserabsorbierenden Baumwolle.

10. Blutgruppendetektionskarte nach Anspruch 9, **dadurch gekennzeichnet, dass** der Festphasenkunststoffträger aus einem Material hergestellt ist, das aus Polyethylen, Polystyrol und Styrol-Butadien-Acrylnitril ausgewählt ist; der Festphasenkunststoffträger ferner ein Detektionsloch umfasst, das zwei Löcher, drei Löcher oder mehrere Löcher umfasst; die isolierende Membran ein Filterpapier, Zellulosefasern, eine Glasfasermatte, eine Fasermatte aus einem porösen hochpolymeren Material oder ein Testpapier umfasst; und die saugfähige Baumwolle eine Baumwollzellstoffmatte und ein Filterpapier umfasst.

11. Verfahren zum Detektieren einer Blutgruppe, umfassend die folgenden Schritte:
Hinzufügen einer zu detektierenden Blutgruppenprobe in das Detektionsloch der Blutgruppendetektionskarte nach einem der Ansprüche 9-10 und Stehenlassen der zu detektierenden Blutgruppenprobe für mindestens 1 Minute; und Spülen mit Waschflüssigkeit, Beobachten der Farbentwicklung des Detektionslochs, wobei eine rote Farbentwicklung ein positives Ergebnis anzeigt und eine natürliche Farbentwicklung ein negatives Ergebnis anzeigt,
wobei ein positives Ergebnis beobachtet wird, wenn die zu detektierende Blutgruppenprobe eine Antigen-Antikörper-spezifische Reaktion mit dem biologischen mehrschichtigen porösen Membran-Immunträger zur Blutgruppenkompatibilitätsdetektion in der Blutgruppendetektionskarte eingeht; und andernfalls das Ergebnis negativ ist.

12. Verwendung des funktionalisierten biologischen mehrschichtigen porösen Membran-Immunträgers nach Anspruch 1 oder 2 bei der Herstellung eines Produkts zur Blutgruppenkompatibilitätsdetektion.

13. Verwendung des funktionalisierten biologischen mehrschichtigen porösen Membran-Immunträgers nach Anspruch 1 oder 2 bei der Herstellung eines Produkts zur Detektion und Analyse eines pathogenen mikrobiellen Zellantigen-Antikörpers, eines bakteriellen Zellantigen-Antikörpers oder eines Tumorzellantigen-Antikörpers.

## Revendications

1. Support immunitaire à membrane poreuse multicouche biologique fonctionnalisé, comprenant :
une membrane poreuse multicouche biologique ;
un anticorps à membrane poreuse multicouche antibiologique spécifique, l'anticorps à membrane poreuse multicouche antibiologique spécifique étant fixé à la surface de la membrane poreuse multicouche biologique par liaison spécifique antigène-anticorps avec la membrane poreuse multicouche biologique ; et
un anticorps réticulé, l'anticorps réticulé étant couplé et lié à l'anticorps à membrane poreuse multicouche antibiologique spécifique,
dans lequel une extrémité Fab de l'anticorps réticulé est capable d'être couplée à l'anticorps à membrane poreuse multicouche antibiologique spécifique, et l'autre extrémité Fab est capable d'être liée à un anticorps spécifique.

2. Support immunitaire à membrane poreuse multicouche biologique fonctionnalisé selon la revendication 1, **caractérisé en ce que** l'anticorps à membrane poreuse multicouche antibiologique spécifique comprend un anticorps monoclonal murin, un anticorps polyclonal dérivé de chèvre, un anticorps monoclonal/anticorps polyclonal dérivé de lapin ou un anticorps polyclonal dérivé de poulet ; l'anticorps réticulé comprend un anticorps polyclonal anti-souris, un anticorps polyclonal anti-chèvre, un anticorps polyclonal anti-lapin ou un anticorps polyclonal anti-poulet ; et l'anticorps spécifique qui est capable d'être lié à l'autre extrémité Fab de l'anticorps réticulé est un anticorps monoclonal murin, un anticorps polyclonal dérivé de chèvre, un anticorps monoclonal/polyclonal dérivé de lapin ou un anticorps polyclonal dérivé de poulet.

3. Procédé de préparation d'un support immunitaire à membrane poreuse multicouche biologique fonctionnalisé, comprenant les étapes suivantes de :
S1 : cisaillement d'un tissu de coton épais nettoyé ou d'un tissu de laine ou d'un tissu de soie naturelle ou d'un tissu de fibres végétales en une membrane ronde ou carrée, puis immersion de la membrane dans une solution de traitement et trempage pendant 2 à 4 heures dans un bain-marie à une température de 40 °C à 70 °C pour préparer une membrane poreuse multicouche biologique ; et
S2 : réalisation d'une liaison spécifique antigène-anticorps de la membrane poreuse multicouche biologique à un anticorps à membrane poreuse multicouche antibiologique spécifique, lavage avec un liquide de lavage, puis couplage avec un anticorps réticulé, lavage avec un liquide de lavage, blocage avec une solution de blocage, et lavage avec un liquide de lavage pour préparer le support immunitaire à membrane poreuse multicouche biologique fonctionnalisé selon la revendication 1,
dans lequel l'anticorps à membrane poreuse multicouche antibiologique spécifique est un anticorps capable de subir une réaction spécifique antigène-anticorps avec la membrane poreuse multicouche biologique ; et
une extrémité Fab de l'anticorps réticulé est capable d'être couplée à l'anticorps à membrane poreuse multicouche antibiologique spécifique, et l'autre extrémité Fab est capable d'être liée à un anticorps spécifique.

4. Support immunitaire à membrane poreuse multicouche biologique pour la détection de la compatibilité de groupes sanguins, comprenant :
le support immunitaire à membrane poreuse multicouche biologique fonctionnalisé selon la revendication 1 ou 2 ; et
un anticorps de groupe sanguin des globules rouges ou un antigène sensibilisé de groupe sanguin des globules rouges, fixé au support immunitaire à membrane poreuse multicouche biologique fonctionnalisé par liaison spécifique avec l'anticorps réticulé sur le support immunitaire à membrane poreuse multicouche biologique fonctionnalisé,
dans lequel l'antigène sensibilisé de groupe sanguin des globules rouges est un antigène de groupe sanguin des globules rouges lié spécifiquement à un anticorps membranaire des globules rouges d'un anticorps de groupe non sanguin.

5. Support immunitaire à membrane poreuse multicouche biologique pour la détection de compatibilité de groupes sanguins selon la revendication 4, **caractérisé en ce que** l'anticorps de groupe sanguin des globules rouges comprend des anticorps de groupe sanguin de type IgM, de type IgG ou de type IgM-IgG mixtes pour les systèmes de groupes sanguins ABO, Rh, MNS, Kell et Kidd, et l'anticorps de groupe sanguin des globules rouges a un titre supérieur à 1:32.

6. Support immunitaire à membrane poreuse multicouche biologique pour la détection de compatibilité de groupes sanguins selon la revendication 4, **caractérisé en ce que** l'antigène sensibilisé du groupe sanguin des globules rouges comprend un antigène sensibilisé des globules rouges de type A, un antigène sensibilisé des globules rouges de type B, un anticorps irrégulier sensibilisé criblant un antigène des globules rouges ou un antigène sensibilisé du groupe des globules rouges.

7. Procédé de préparation d'un support immunitaire à membrane poreuse multicouche biologique pour la détection de compatibilité de groupes sanguins, comprenant les étapes suivantes de :
S1 : cisaillement d'un tissu de coton épais nettoyé ou d'un tissu de laine ou d'un tissu de soie naturelle ou d'un tissu de fibres végétales en une membrane ronde ou carrée, puis immersion de la membrane dans une solution de traitement et trempage pendant 2 à 4 heures dans un bain-marie à une température de 40 °C à 70 °C pour préparer une membrane poreuse multicouche biologique ;
S2 : réalisation d'une liaison spécifique antigène-anticorps de la membrane poreuse multicouche biologique à un anticorps à membrane poreuse multicouche antibiologique spécifique, lavage avec un liquide de lavage, puis couplage avec un anticorps réticulé, lavage avec un liquide de lavage, blocage avec une solution de blocage, et lavage avec un liquide de lavage pour préparer le support immunitaire à membrane poreuse multicouche biologique fonctionnalisé selon la revendication 1 ; et
S3 : réalisation d'une liaison spécifique d'un anticorps de groupe sanguin des globules rouges ou d'un antigène sensibilisé du groupe sanguin des globules rouges à l'anticorps réticulé sur le support immunitaire à membrane poreuse multicouche biologique fonctionnalisé pour préparer le support immunitaire à membrane poreuse multicouche biologique pour la détection de la compatibilité de groupes sanguins de la revendication 4, dans lequel l'anticorps à membrane poreuse multicouche antibiologique spécifique est un anticorps capable de subir une réaction spécifique antigène-anticorps avec la membrane poreuse multicouche biologique ;
une extrémité Fab de l'anticorps réticulé est capable d'être couplée à l'anticorps à membrane poreuse multicouche antibiologique spécifique, et l'autre extrémité Fab est capable d'être liée à un anticorps spécifique, et l'anticorps spécifique est un anticorps de groupe sanguin des globules rouges ou un antigène sensibilisé de groupe sanguin des globules rouges capable d'être lié à l'extrémité Fab de l'anticorps réticulé ; et
l'antigène sensibilisé du groupe sanguin des globules rouges est un antigène de groupe sanguin des globules rouges spécifiquement lié à un anticorps à membrane de globules rouges d'un anticorps de groupe non sanguin.

8. Procédé de préparation du support immunitaire à membrane poreuse multicouche biologique pour la détection de compatibilité de groupes sanguins selon la revendication 7, comprenant en outre une étape de préparation de l'antigène sensibilisé du groupe sanguin des globules rouges comme suit : réaction de l'anticorps à membrane des globules rouges de l'anticorps non sanguin avec des globules rouges frais, lyse des globules rouges par une solution hypotonique lorsque l'intensité d'agglutination atteint 1+, centrifugation et collecte pour obtenir l'antigène sensibilisé des globules rouges.

9. Carte de détection de groupe sanguin, comprenant :
un support en plastique en phase solide, comprenant une enveloppe extérieure à rainure de serrage et une enveloppe intérieure à rainure de serrage, l'enveloppe extérieure à rainure de serrage et l'enveloppe intérieure à rainure de serrage étant reliées de manière amovible ;
le support immunitaire à membrane poreuse multicouche biologique pour la détection de compatibilité de groupes sanguins selon l'une quelconque des revendications 4 à 6, positionné au fond de l'enveloppe extérieure à rainure de serrage du support en plastique en phase solide ;
un coton absorbant, placé au fond de l'enveloppe intérieure à rainure de serrage du support en plastique en phase solide ; et
une membrane isolante, agencée au-dessus du coton absorbant l'eau.

10. Carte de détection de groupe sanguin selon la revendication 9, **caractérisée en ce que** le support en plastique en phase solide est fait d'un matériau choisi parmi le polyéthylène, le polystyrène et le styrène-butadiène-acrylonitrile ; le support en plastique en phase solide comprend en outre un trou de détection, comprenant deux trous, trois trous ou plusieurs trous ; la membrane isolante comprend un papier filtre, des fibres de cellulose, un mat de fibres de verre, un mat de fibres fait d'un matériau hautement polymérique poreux ou un papier test ; et le coton absorbant comprend un mat de pâte de coton et un papier filtre.

11. Procédé de détection d'un groupe sanguin, comprenant les étapes suivantes de :
ajout d'un échantillon de groupe sanguin à détecter dans le trou de détection de la carte de détection de groupe sanguin selon l'une quelconque des revendications 9 et 10, et le fait de laisser reposer l'échantillon de groupe sanguin à détecter pendant au moins 1 minute ; et rinçage avec un liquide de lavage, observation du développement d'une couleur du trou de détection, dans lequel le développement d'une couleur rouge indique un résultat positif, et un développement d'une couleur naturelle indique un résultat négatif, dans lequel un résultat positif est observé si l'échantillon de groupe sanguin à détecter subit une réaction spécifique antigène-anticorps avec le support immunitaire à membrane poreuse multicouche biologique pour la détection de compatibilité de groupes sanguins dans la carte de détection de groupe sanguin ; sinon, le résultat est négatif.

12. Utilisation du support immunitaire à membrane poreuse multicouche biologique fonctionnalisé selon la revendication 1 ou 2 dans la préparation d'un produit pour la détection de la compatibilité de groupes sanguins.

13. Utilisation du support immunitaire à membrane poreuse multicouche biologique fonctionnalisé selon la revendication 1 ou 2 dans la préparation d'un produit pour détecter et analyser un anticorps d'antigène de cellule microbienne pathogène, un anticorps d'antigène de cellule bactérienne ou un anticorps d'antigène de cellule tumorale.
